# EUROPEAN PATENT SPECIFICATION

(11) **EP 1 815 013 B1**
(45) Date of publication and mention of the grant of the patent: **29.12.2010**
(21) Application number: 05810094.2
(22) Date of filing: 22.11.2005
(51) Int. Cl.: C12Q 1/68

(54) **COMPOSITION FOR AMPLIFYING NUCLEIC ACIDS**
ZUSAMMENSETZUNG ZUR VERSTÄRKUNG VON NUKLEINSÄUREN
COMPOSITION POUR L AMPLIFICATION D ACIDES NUCLEIQUES

(30) Priority: 22.11.2004 FR 0412395
(43) Date of publication of application: 08.08.2007
(73) Proprietor: Bio-Rad Pasteur, 92430 Marnes-la-Coquette (FR)
(72) Inventor: SAVOYE, Chantal, Sainte-Foy, Quebec G1X4C4 (CA); SARFATI, Patrice, F-92380 Garches (FR)
(74) Representative: Colombet, Alain André
(86) International application number: PCT/IB2005/003503
(87) International publication number: WO 2006/054172

(56) References cited:
- EP-A- 0 678 581
- EP-A- 0 726 310
- EP-A- 0 915 173
- WO-A-97/11196
- US-A- 5 846 701
- US-A1- 2002 009 738
- US-A1- 2004 121 338
- KOONJUL PRIYUM K ET AL: "Inclusion of polyvinylpyrrolidone in the polymerase chain reaction reverses the inhibitory effects of polyphenolic contamination of RNA" NUCLEIC ACIDS RESEARCH, vol. 27, no. 3, 1 February 1999 (1999-02-01), pages 915-916, XP002348231 ISSN: 0305-1048

## Description

The invention relates to a stable composition of reagents for amplifying nucleic acids.

The amplification of nucleic acids is commonly used for detecting small amounts of specific genes in the diagnostic field, or medical field in particular.

Several amplification techniques exist: PCR (polymerase chain reaction), RT-PCR (reverse transcription followed by PCR), SDA (strand displacement amplification), NASBA (nucleic acid sequence-based amplification), etc. Each one of them requires the use of a mixture of several reagents: one or more oligonucleotide primers, dNTPs (deoxynucleotide triphosphates), one or more enzymes (polymerase, reverse transcriptase, etc.), a saline buffer and, advantageously, one or more fluorescent probes.

The mixing of these reagents involves meticulous pipetting of each of them, in order to achieve the required sensitivity and specificity performance levels and to ensure reproducibility of the amplification results. In addition, the preparation of the mix of amplification reagents must be physically separated from the addition of the nucleic acid to be amplified so as to prevent contamination of the stock solutions of amplification reagents with the nucleic acid to be amplified.

These reagents are generally conserved at -20°C, and the probes and primers are most commonly lyophilized so as to ensure long-lasting storage.

Cryoprotective agents may then be used, for example sugars or polyols, so as to maintain the integrity of the oligonucleotides during lyophilization (EP 833 667). Similarly, glycerol is used to protect the enzymes (EP 455 744).

EP 455 744 provides, moreover, a reaction concentrate for sequencing nucleic acids, which comprises a thermostable polymerase, dNTPs, a ddNTP, a reducing agent, glycerol, and, optionally, a primer, thus avoiding mixing of these reagents at the time of use.

Moreover, amplification kits containing all the reagents required for the amplification are sold by the company ARTUS. However, these kits must imperatively be conserved at -20°C. Furthermore, the supplier indicates that conservation at 4°C cannot exceed 5 hours.

No composition, stable for several months at 4°C, for amplifying nucleic acids (commonly called a "mix"), containing all the reagents required for the amplification reaction, and in particular one or more fluorescent nucleotide probes, exists.

The use of fluorescent nucleotide probes is particularly advantageous for detecting and monitoring amplification reactions (such as, in particular, real-time PCR or RT-PCR). This is because amplification tests using fluorescent probes eliminate any manual post-amplification step for detection, which makes it possible to obtain a rapid result and prevent the risk of contaminations with aerosols ("carryover").

Now, the problem that arises is that of the stability of the fluorescent probes used, which degrade rapidly, adding to the instability of the enzymes and of the dNTPs used for the amplification.

The authors of the present invention have endeavoured to solve these problems while at the same time wishing to avoid the drawbacks of extemporaneous mixing of the reagents.

The authors of the invention have then demonstrated that the presence of polyvinylpyrrolidone (PVP) and optionally of a polyol makes it possible to stabilize both the enzymes and the fluorescent probes in a liquid composition, or even also the dNTPs.

A subject of the invention is therefore a concentrated and buffered liquid composition for amplifying nucleic acids, comprising at least one dNTP, at least one enzyme required for the amplification, at least one nucleotide primer, and at least one fluorescent nucleotides probe, in the presence of polyvinylpyrrolidone (PVP) and optionally of a polyol. In particular, when the buffered liquid composition for amplifying nucleic acids comprises both a polyol and PVP, the polyol is preferably selected from the group consisting of glycerol, sorbitol, pentaerythritol, inositol, dulcitol, mannitol, propylene glycol or ethylene glycol.

Such a composition, also commonly referred to as "mix", offers the advantage of being stable over time independently of the storage conditions. Even after conservation for several months art ambient temperature, the enzymes, the fluorescent probes, or even also the dNTPs, remain stable. The level of sensitivity and specificity required for the amplification and the detection of the nucleic acid being ensured.

The composition does not require the use of any additional handling other than the addition of a diluent, and, for this reason, can be considered as a ready-to-use reaction mix.

This composition is useful regardless of the type of nucleic acid amplification envisaged.

The term "amplification" is intended to mean the increase in concentration of a specific nucleic acid sequence from a purified nucleic acid or from a mixture of nucleic acid sequences. This amplification step can be carried out by any conventional method of enzymatically amplifying DNA or RNA, such as, in particular, PCR described by Saiki et al. (1988) and in patents EP 200 362 and 201 184, the TAS (transcription-based amplification system) techniques proposed by Kwoh et al. (1989), the 3SR (self-sustained sequence replication) technique described by Fahy et al. (1991), the NASBA (nucleic acid sequence-based amplification) technique described in EP 329 822, the transcription mediated amplification (TMA) technique described in US 5,399,491, the SDA (strand displacement amplification) technique described by Walker et al. (1992), the ligase chain reaction (LCR, gap-LCR) technique described in patent EP 0 320 308, the rolling circle amplification (RCA) technique described in Nat. Genet. (1998) Jul; 19(3):225-232 or else the LLA (linked linear amplification) technique described in patent US 6,027,923.

Preferably, the amplification is a real-time PCR or a real-time RT-PCR.

A preferred composition is a composition as defined above for amplifying nucleic acids by PCR, comprising dATP, dCTP, dGTP and one from dTTP or dUTP, and also at least one enzyme required for the PCR, at least two oligonucleotide primers, and at least one fluorescent nucleotide probe, in the presence of PVP and optionally of a polyol.

Another preferred composition is a composition as defined above for amplifying nucleic acids by RT-PCR, comprising dATP, dCTP, dGTP, and one from dTTP or dUTP, and also at least one enzyme required for the RT-PCR, at least two oligonucleotide primers, and at least one fluorescent nucleotide probe, in the presence of PVP and optionally of a polyol.

### Buffer:

The term "buffered composition" is intended to mean that the pH of the composition is controlled by the presence of a buffer. It may be a standard buffered saline solution, for example containing a tris-(hydroxymethyl)aminomethane (TRIS®) salt, preferably the hydrochloride or the acetate, in water so as to attain a concentration of TRIS® of 5 mM to 500 mM, preferably of 7 mM to 400 mM.

A magnesium and/or manganese salt (preferably chloride or sulphate) can be added to this buffer solution so as to provide a working concentration of 1 mM to 8 mM, preferably of 1.5 to 5 mM. A potassium salt, preferably potassium chloride, can also be added to the solution, at a working concentration of 5 mM to 1 M, preferably of 7 mM to 800 mM. A sodium salt, preferably sodium chloride, can also be added, at a concentration of 5 mM to 500 mM, preferably of 7 mM to 400 mM. An ammonium salt, for example ammonium sulphate, can also be added to the mixture at a working concentration of 2 mM to 100 mM, preferably 3.5 mM to 80 mM. Combinations of ammonium sulphate and of potassium chloride, or of other salts, can also be used at concentrations equivalent to those already mentioned. Other compounds can also be added, such as ethylenediaminetetraacetate (EDTA), dithiothreitol (DTT), tween-20, BSA (bovine serum albumin) or triton X-100. The pH is adjusted so as to obtain a pH value of between 7.4 and 9.2, preferably 7.8 and 8.8.

### Enzymes:

The composition comprises at least one enzyme required for the amplification. It may be a DNA polymerase, such as *Taq* DNA polymerase, VENT™, DEEPVENT™, Pfu, Pwo or Tth. It may also be "hot start" Taq polymerase, the enzymatic site of which can be blocked at low temperature by means of an immunoreaction (EP 592 035), a chemical reaction (US 5,677,152; US 5,773,258) or by means of an ionic interaction. All these enzymes are commercially available. Where appropriate, reverse transcriptases can be used, for example reverse transcriptases with RNase H activity, Other enzymes may be added, for example UDG (uracyl DNA glycosylase). The thermolabile UDG enzyme can in particular be used.

The enzymes, for example the Taq DNA polymerase, the reverse transcriptase and the UDG, are preferably used at concentrations of between 0.5 U and 8U of Taq polymerase per reaction, which corresponds to 10 U/ml to 8 × 10³ U/ml. The expression of the concentration of the enzyme is well known to the man skilled in the art, one unit being the amount of enzyme that incorporates 10 nmol of dNTP into a material insoluble in acid in a period of 30 minutes at 72°C. The reverse transcriptase is preferably used at between 1 U and 10³ U per reaction, which corresponds to 20 U/ml to 10⁶ U/ml. The reverse transcriptase unit is the amount of enzyme that incorporates one nmol of dTTP into products insoluble in acid in 10 minutes at 37°C with a poly-A RNA template and an oligo-dT₁₂₋₁₈ primer. The UDG, when it is added to the composition, is preferably used at between 0.1 and 2 U, corresponding to 2 U/ml to 2 × 10³ U/ml. One unit of UDG catalyses the release of one nmol of free uracil from poly(dU) in one hour at 37°C.

### dNTPs:

The deoxynucleotide triphosphates (dNTPs) are provided at a concentration preferably at least five times greater than the working concentration. This means that each dNTP is preferably used at between 0.3 mM and 75 mM. The dNTPs include deoxyadenosine triphosphate (dATP), deoxyguanosine triphosphate (dGTP), deoxycytidine triphosphate (dCTP) and deoxythymidine triphosphate (dTTP). Preferably, these four dNTPs (dATP, dCTP, dGTP, dTTP) are used in the compositions of the invention. Other dNTPs, such as deoxyuridine triphosphate (dUTP), and dNTP analogues, along with dNTP conjugates, can also be used and are included in the term "dNTP" used here.

### Primers:

Typically, the oligonucleotide primers are generally between 12 and 25 nucleotides in length. However, primers less than 12 nucleotides or greater than 25 nucleotides in length can also be used. The length of the primer is not essential for implementing the invention. Generally, the oligonucleotide primers are synthesized chemically. The oligonucleotide primers can be composed of the bases A, T, G, C or U or base analogues, for example inosine or PNAs (peptide nucleic acids).

The oligonucleotide primers used hybridize conventionally to the complementary strand of the template during the amplification reaction.

Each primer is preferably used at a concentration of at least 5 times the working concentration, which means a concentration of approximately 0.1 µM to 200 µM, preferably of between 1 µM and 50 µM.

### Probes:

The nucleotide probe may comprise from 8 to 40 nucleotides in length. The length of the probe is not essential to the use of the invention. It is typically used to capture or detect a target sequence to which it hybridizes.

Labelling of the probe is particularly advantageous for facilitating the detection of the amplified nucleic acid, in particular in real-time amplification and detection reactions, i.e. the target sequence is detected and/or quantified while the amplification reaction is taking place.

The probe is referred- to as "fluorescent" in that it carries a fluorescent label.

These fluorescent labels include in particular fluorescein, Tamra, N,N,N',N'-tetramethyl-6-carboxyrhodamine; FAM, 5-carboxyfluorescein; JOE, 2',7'-dimethyl-4',5'-dichloro-6-carboxyfluorescein, ROX, 6-carboxy-X-rhodamine; CY3; CY5; TET, tetrachlorofluorescein or HEX, hexachlorofluorescein.

The detection of the amplified nucleic acid can in particular be carried out using the "molecular beacon" technology (Tyagi and Kramer, 1996; Cayouette et al., 1999). According to this technology, a fluorophore and a "quencher" are attached to each end of the sequence of the probe. In the absence of the target nucleic acid, the sequences of the arms hybridize to one another so as to form a "hairpin" structure which brings the fluorophore into contact with the quencher. In the presence of a target nucleic acid, the probe and the target sequence hybridize. The hairpin structure cannot coexist with the rigid double helix that is formed by this hybridization and the conformational change resulting therefrom leads to separation of the sequences of the arms, causing distancing of the fluorophore and of the quencher. When the fluorophore and the quencher are separated, the fluorophore signal is detectable. All the fluorescent labels indicated above can be used. The quencher can preferably be selected from Dabcyl, Eclipse Dark Quencher, and Black Hole Quenchers. These molecules are readily available from Eurogentec, Biosearch Technology, Proligo.

Each fluorescent probe is preferably used at a concentration greater than at least 5 times the working concentration, which means between 0.1 µM and 200 µM, preferably between 0.5 µM and 30 µM.

### Polyols:

The polyols that can be used in the invention have a linear, branched or cyclic structure. The most suitable polyols include glycerol, sorbitol or pentaerythritol. Other polyols, such as inositol, dulcitol, mannitol, propylene glycol or ethylene glycol, and derivatives thereof, can also be used. Combinations of polyols can also be used.

The polyols are preferably dissolved in a buffer or in water, and preferably used in the composition at a concentration of greater than 1 mM, preferably greater than 250 mM, preferably greater than 500 mM, even more preferably greater than 1 M.

Preferably, glycerol is used at a concentration of greater than 5 M, sorbitol is used at a concentration of greater than 1 M, inositol is used at a concentration of greater than 500 mM, and pentaerythritol is used at a concentration of greater than 250 mM.

### PVP:

Polyvinylpyrrolidone is a polymer of formula:

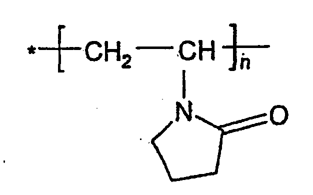

PVP is an excipient in the preparation of pharmaceutical products, mainly for the production of tablets or granules. PVPs with an average molecular weight of 2500 to 750 000 are on the market under the following names: Kollidon, Luviskol, Albigen A, and Divergan (BASF); PVP and Plasdone (General Aniline and Film Corp.); Collacral and Luviskol VA (copolymers with vinyl esters, BASF); PVP/VA (copolymers with vinyl acetate, General Aniline and Film Corp.).

The PVP is preferably dissolved in a buffer or in water. It is used at a concentration of greater than 0.1 mM, in particular at a concentration from 0.1 mM to 5 M, more particularly at a concentration from 1 mM to 1 M, and even more particularly at a concentration from 10 mM to 500 mM.

In the context of the invention, use is preferably made of PVP with a molecular weight of 2500 g/mol to 750 000 g/mol, more preferably use is made of PVP with a molecular weight of 7500 g/mol to 55 000 g/mol, and even more preferably use is made of PVP-10, the molecular weight of which is 10 000 g/mol. The PVP-10 is preferably used at a concentration of greater than 30 mM, preferably at a concentration from 30 mM to 1 M, more preferably at approximately 300 mM.

### Use:

The composition contains, in concentrated form, the reagents required for a nucleic acid amplification. The only additional step required for starting up the amplification is the dilution of this composition before or after it has been brought into contact with the nucleic acid sample to be amplified.

The diluent is preferably a buffered saline solution, optionally comprising magnesium salts or manganese salts, among other salts. The buffer and salt concentrations are adjusted so as to obtain final buffer and salt concentrations that are suitable for an amplification reaction. It may be a buffered saline solution that contains at least one of the ingredients selected from TRIS at a concentration of at least 8 mM, potassium salt or sodium salt at a concentration of at least 8 mM, ammonium salt at a concentration of at least 5 mM, and magnesium salt or manganese salt at a concentration of at least 0.8 mM, the ingredients being used alone or as a mixture.

It may, for example, be a standard buffered saline solution, containing a tris(hydroxymethyl)aminomethane (TRIS) salt, preferably the hydrochloride or the acetate, in water, so as to attain a TRIS concentration of 8 mM to 2.5 M, preferably of 10 mM to 125 mM. A magnesium salt and/or manganese salt, preferably chloride or sulphate, can be added to this buffer solution at a concentration of 0.85 mM to 400 mM, preferably of 1 mM to 20 mM. A potassium salt, preferably potassium chloride can also be added to the solution, at a working concentration of 8 mM to 4.5 M, preferably of 10 mM to 250 mM. A sodium salt, preferably chloride, can also be added, at a concentration of 8 mM to 2.5 M, preferably of 10 mM to 125 mM. An ammonium salt, for example ammonium sulphate, can also be added to the mixture at a working concentration of 5 mM to 500 mM, preferably 5 mM to 25 mM. Combinations of ammonium sulphate and of potassium chloride, or of other salts, can also be used at concentrations equivalent to those mentioned above. Other compounds can also be used, such as ethylenediaminetetraacetate (EDTA), dithiothreitol (DTT), tween-20, BSA (bovine serum albumin) or triton X-100. The pH is adjusted so as to obtain a pH value of between 7.4 and 9.2, preferably 7.8 and 8.8.

An example of a diluent is a buffer for *Taq* DNA polymerase at an MgCl₂ concentration of between 1 mM and 20 mM.

Several embodiments are possible for the preparation of a complete reaction mix for nucleic acid amplification.

A preferred aspect of the invention is directed towards a method of preparing a complete reaction mix for amplifying nucleic acids, comprising:
(i) diluting a concentrated composition as defined above, in a buffered saline solution suitable for amplifying nucleic acids;
(ii) bringing a sample of nucleic acids to be amplified into contact with a desired volume of the composition diluted in step (i).

Preferably, the method of preparation comprises the following steps:
(i₁) dispensing the concentrated composition into a container and storing in this form,
(i₂) adding a buffered saline solution at the time of use,
(ii) bringing a sample of nucleic acids to be amplified into contact with the composition diluted in step (i₂).

In practice, it is possible, for example, to take 5 µl of a concentrated composition as defined above (mix), and to add 35 µl of diluent (buffer) and then 10 µl of a nucleic acid sample, or else to take 5 µl of the concentrated composition (mix), and to add 20 µl of diluent (buffer) in 25 µl of sample.

The bringing into contact can be carried out in any container suitable for the amplification, such as tubes, wells of a microplate, capillaries.

Alternatively, another method of preparing a complete reaction mix for amplifying nucleic acids, comprises:
(i) mixing a sample of nucleic acids to be amplified, in a buffered saline solution suitable for amplifying nucleic acids;
(ii) adding the mix obtained in step (i) to a desired volume of the concentrated composition as defined above.

Preferably, this method comprises, in practice, dispensing the concentrated composition as defined above into tubes, into the wells of a microplate, or into any other container suitable for the amplification, and then distributing into the concentrated composition the nucleic acid sample (mixed beforehand with the buffered saline solution).

The amplification can start spontaneously or after a hot (95°C) incubation, for example, if a "hot start" polymerase is used.

The nucleic acid sample may be of any type. It preferably comes from a biological sample such as blood, urine, saliva or a tissue biopsy, which, advantageously, has been treated beforehand, so as to extract nucleic acids therefrom, by any method known to those skilled in the art.

The present invention is therefore also directed towards a method of amplifying nucleic acids, comprising steps (i) and/or (ii) as defined above, and (iii) the starting of the reaction for amplifying the nucleic acids present in the sample, which amplification is detectable by means of the fluorescent label carried by the probe(s).

It may be any type of amplification, as described above, for example a real-time RT-PCR or a real-time PCR.

### Kits:

The invention also provides a kit for amplifying and/or detecting nucleic acids, comprising at least one container containing the concentrated composition as defined above and, optionally, instructions for use of the kit. Other kits may comprise a first container containing the concentrated composition, as defined above, and a second container containing a buffered saline solution as defined above.

### FIGURE LEGENDS:

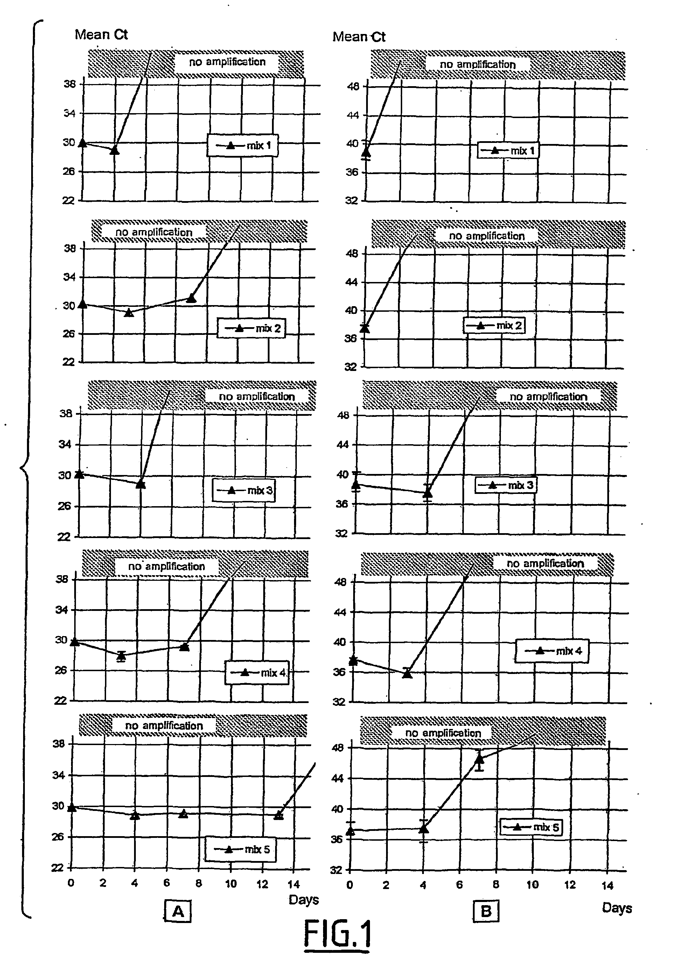

**Figure 1****:** Comparison of the Ct values obtained for the amplification of 1000 copies of DNA (A) and 10 copies of DNA (B) after various incubation times, at 37°C, of the PCR mixes containing no polyol (mix 1), 6.5 M glycerol (mix 2), 750 mM inositol (mix 3), 530 mM pentaerythritol (mix 4), 1.5 M sorbitol (mix 5).

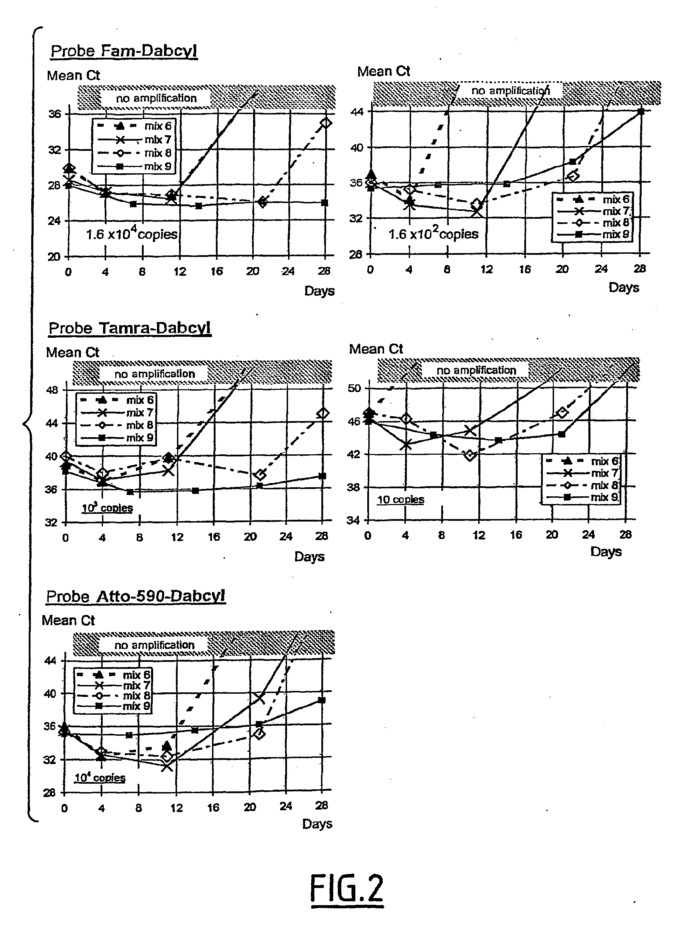

**Figure 2****:** Comparison of the Ct values obtained from the amplification of 3 DNA sequences, after various incubation times, at 37°C, of the PCR mixes containing neither a polyol nor PVP (mix 6), or containing 0.3 M PVP10 (mix 7), 6.5 M glycerol (mix 8), 0.3 M PVP-10 + 6.5 M glycerol (mix 9).

### EXAMPLES:

### Example 1:

This example shows the stabilizing effect of glycerol, of sorbitol, of pentaerythritol and of inositol on mixtures for real-time PCR.

The study of the reagent stability is carried out at 37°C. Based on Arrhenius' law, it is accepted that an enzyme that is stable for one week at 37°C conserves its activity for 6 months at 4°C.

Five compositions for real-time PCR are prepared. Each composition (mix) contains 1X buffer (Qiagen), 1.5 mM of MgCl₂, 5 µM of each primer (Eurobio Laboratoires), 2.5 mM of each dNTP (Eurobio Laboratoires), 1 µM of Molecular Beacon probe labelled with Fam-Dabcyl (Eurogentec), 1 U of Taq polymerase (Qiagen) and
no polyol: mix 1,
6.8 M of glycerol (Prolabo): mix 2,
750 mM inositol (Fluka): mix 3,
530 mM of pentaerythritol (Merck): mix 4,
1.5 M sorbitol (Sigma): mix 5.

Aliquot fractions of 45 µl are taken from each composition (mix) and conserved at 37°C until the date of analysis.

A diluent solution is prepared. It contains 1.29X buffer and 6.93 mM of MgCl₂. Aliquot fractions of 315 µl are taken and conserved at 37°C until the date of analysis.

On the day of analysis, an aliquot fraction of the diluent is added to an aliquot fraction of the PCR mix. After mixing, 40 µl of the solution are dispensed into the PCR tubes. 10 µl of *Mycobacterium tuberculosis* DNA containing 1 copy/µl are added to three different PCR tubes, 1.0 µl of *Mycobacterium tuberculosis* DNA containing 100 copies/µl are added to three different tubes, and 10 µl of water are added to two different tubes.

PCR reactions are carried out on day 0, on day 3 ± 1, on day 6 or 7, on day 13 and on day 21.

The PCR reactions are carried out on the iQ icycler (Bio-Rad). 50 cycles made up of 30 seconds at 94°C, 30 seconds at 59°C and 30 seconds at 72°C are carried out, after an initial step of 15 minutes at 95°C in order to activate the "hot start" polymerase.

The analyses are carried out by means of the iQ icycler software. The cycle threshold (Ct) values for each PCR reaction are determined after manual positioning of the baseline between cycles 4 and 25 and manual positioning of the threshold at a constant value of 50 relative fluorescence units (RFU). The Ct is the cycle of the PCR from which the fluorescence measured is greater than that of the background noise.

The means of the Ct values for the triplicates obtained after amplification of 10 copies/PCR and 1000 copies/PCR with the mixes (mixes 1 to 5) incubated at 37°C are shown in figure 1.

All the polyols presented in this example make it possible to stabilize all the reaction mixes for real-time PCR. Pentaerythritol and sorbitol are the most effective as stabilizers since they increase the stability of the mixes by a factor at least equal to 3.5 and 6.5, respectively.

Mixes 3, 4, 5 and 5' were further tested for their stability at three temperatures (37°C, 4°C, -20°C). Mix 5' is identical to mix 5 except for MgCl₂ concentration (3 mM) and probe concentration (2 µM). The results obtained for mixes 3, 4, 5 and 5' are respectively presented in Tables 1A, 1B, Tables 2A, 2B, Tables 3A, 3B, and Tables 4A, 4B below.

**Tables 1A and 1B: Comparison of the Ct values obtained for the amplification of 1000 copies of DNA (A) and 10 copies of DNA (B) after various incubation times, at 37°C, 4°C and -20°C, of the PCR mix 3 containing 750 mM inositol:**

| | **Ct (1000 copies / PCR)** | | |
|---|---|---|---|
| | **37°C** | **4°C** | **-20°C** |
| **D0** | 30.3 ± 0,3 | 30.3 ± 1,3 | 30.3 ± 1.3 |
| **D4** | 29.0 ± 0,1 | 29.6 ± 0,3 | NT |
| **D7** | > 50 | NT | NT |
| **10 months** | NT | 30.5 ± 0.6 | 31.7 ± 1.4 |
| | | | |

| | **Ct (10 copies / PCR)** | | |
|---|---|---|---|
| | **37°C** | **4°C** | **-20°C** |
| **D0** | 38.6 ± 1.3 | 38.6 ± 1.3 | 38.6 ± 1.3 |
| **D4** | 37.5 ± 1.1 | NT | NT |
| **D7** | > 50 | NT | NT |
| **10 months** | NT | 38.3 ± 0.6 | 39.2 ± 4.1 |

**Tables 2A and 2B: Comparison of the Ct values obtained for the amplification of 1000 copies of DNA (A) and 10 copies of DNA (B) after various incubation times, at 37°C and 4°C, of the PCR mix 4 containing 530 mM pentaerythritol:**

| | **Ct (1000 copies / PCR)** | |
|---|---|---|
| | **37°C** | **4°C** |
| **D0** | 29.9 ± 0.2 | 29.9 ± 0.2 |
| **D3** | 28.0 ± 0.7 | NT |
| **D7** | 29.3 ± 0.2 | NT |
| **D14** | > 50 | NT |
| **10 months** | NT | 30.0 ± 0.4 |
| | | |

| | **Ct (10 copies / PCR)** | |
|---|---|---|
| | **37°C** | **4°C** |
| **D0** | 37.7 ± 0.1 | 37.7 ± 0.1 |
| **D3** | 35.8 ± 0.7 | NT |
| **D7** | > 50 | NT |
| **D14** | > 50 | NT |
| **10 months** | NT | 37.5 ± 1.0 |

**Tables 3A and 3B: Comparison of the Ct values obtained for the amplification of 1000 copies of DNA (A) and 10 copies of DNA (B) after various incubation times, at 37°C, 4°C and -20°C, of the PCR mix 5 containing 1.5 M sorbitol and 1.5 mM MgCl₂:**

| | **Ct (1000 copies / PCR)** | | |
|---|---|---|---|
| | **37°C** | **4°C** | **- 20°C** |
| **D0** | 29.9 ± 0.1 | 29.9 ± 0.1 | 29.9 ± 0.1 |
| **D4** | 28.9 ± 0.3 | NT | NT |
| **D7** | 29.2 ± 0.5 | NT | NT |
| **D13** | 29.0 ± 0.1 | NT | NT |
| **D21** | > 50 | NT | NT |
| **6 months** | NT | 30.2 ± 0.1 | 31.3 ± 0.6 |
| **12 months** | NT | 29.8 ± 0.1 | 29.2 ± 0.7 |
| | | | |

| | **Ct (10 copies / PCR)** | | |
|---|---|---|---|
| | **37°C** | **4°C** | **- 20°C** |
| **D0** | 37.2 ± 0.9 | 37.2 ± 0.1 | 37.2 ± 0.1 |
| **D4** | 37.5 ± 1.5 | 37.3 ± 0.2 | NT |
| **D7** | 46.6 ± 1.4 | NT | NT |
| **D13** | > 50 | NT | NT |
| **D21** | > 50 | NT | NT |
| **6 months** | NT | 37.8 ± 0.8 | 39 ± 0.7 |
| **12 months** | NT | 37.5 ± 1.0 | 37.1 ± 1.1 |

**Tables 4A and 4B: Comparison of the Ct values obtained for the amplification of 1000 copies of DNA (A) and 10 copies of DNA (B) after various incubation times, at 37°C, 4°C and -20°C, of the PCR mix 5' containing 1.5 M sorbitol and 3 mM MgCl₂:**

| | **Ct (1000 copies / PCR)** | | |
|---|---|---|---|
| | **37°C** | **4°C** | **- 20°C** |
| **D0** | 29.2 ± 0.2 | 29.2 ± 0.2 | 29.2 ± 0.2 |
| **D4** | 29.2 ± 0.3 | NT | NT |
| **D7** | 27.4 ± 0.4 | NT | NT |
| **D14** | 29.4 ± 0.3 | NT | NT |
| **D21** | 28.8 ± 0.1 | NT | NT |
| **D28** | 29.0 ± 0.3 | NT | NT |
| **6 months** | NT | 28.8 ± 0.3 | 31.3 ± 0.6 |
| **12 months** | NT | 27.97 ± 0.2 | 29.2 ± 0.7 |
| | | | |

| | **Ct (10 copies /PCR)** | | |
|---|---|---|---|
| | **37°C** | **4°C** | **-20°C** |
| **D0** | 36.4 ± 0.4 | 36.4 ± 0.4 | 36.4+0.4 |
| **D4** | 37.6 ± 1.1 | NT | NT |
| **D7** | 35.2 ± 0.8 | NT | NT |
| **D14** | 40.7 ± 1 | NT | NT |
| **D21** | 39.5 ± 2.4 | NT | NT |
| **D28** | > 50 | NT | NT |
| **6 months** | NT | 35.6 ± 0.7 | 38.9 ± 0.7 |
| **12 months** | NT | 38.25 ± 1.8 | 37.1 ± 1.1 |

As can be seen, the polyols presented in this example make it possible to stabilize all the reaction mixes for real-time PCR for more than 10 months at 4°C or -20°C.

### Example 2:

This example demonstrates the stabilizing effect of glycerol and of PVP-1 0 on mixes for real-time PCR.

Four concentrated compositions for real-time PCR are prepared. Each composition ("mix") contains 1X buffer (Quiagen), 5 mM of MgCl₂, 5 µM of each primer (Eurobio Laboratoires), 2.5 mM of each dNTP (Eurobio Laboratoires), 2 µM of Molecular Beacon probe labelled with Fam-Dabcyl (Eurogentec), 4 µM of Molecular Beacon probe labelled with Tamra-Dabcyl (Eurogentec), 2 µM of Molecular Beacon probe labelled with Atto-590-Dabcyl (Eurogentec), 2 U of Taq polymerase (Qiagen) and
- neither PVP nor a polyol: mix 6,
- 0.3 M PVP-10 (sigma): mix 7,
- 6.5 M of glycerol (Prolabo): mix 8
- 6.5 M of glycerol and 0.3 M of PVP-1 0: mix 9
- 5 M of glycerol and 0.3 M of PVP-10: mix 10.

Three sequences are amplified and detected simultaneously. Sequences 1 and 2 belong to the genome of *Mycobacterium tuberculosis* and sequence 3 is an internal standard.

Sequence 1 is detected by the Fam probe, sequence 2 is detected by the Tamra probe and sequence 3 is detected by the Atto-590 probe.

Aliquot fractions of 45 µl are taken from each composition (mix) and conserved at 37°C until the date of analysis.

A diluent solution is prepared. It contains 1.15X buffer and 5.75 mM of MgCl₂. Aliquot fractions of 315 µl are taken and conserved at 37°C until the date of analysis.

On the day of analysis, an aliquot fraction of the diluent is added to an aliquot fraction of the PCR mix. After mixing, 40 µl of the solution are dispensed into the PCR tubes.

10 µl of *Mycobacterium tuberculosis* DNA containing 1 copy/µl of sequence 2 are added to two different PCR tubes, 10 µl of *Mycobacterium tuberculosis* DNA containing 100 copies/µl of sequence 2 are added to two different tubes, and 10 µl of water are added to two different tubes.

PCR reactions were carried out on day 0, on day 3 ± 1, on day 6 or 7, on day 13 and on day 21.

The PCR reactions are carried out on the iQ icycler (Bio-Rad). 50 cycles made up of 30 seconds at 94°C, 30 seconds at 59°C and 30 seconds at 72°C are carried out, after an initial step of 15 minutes at 95°C in order to activate the "hot start" polymerase.

The analyses are carried out by means of the iQ icycler software.

The Ct values for each PCR reaction corresponding to sequence 1 (Fam-Dabcyl probe) are determined after manual positioning of the baseline between cycles 4 and 23 and manual positioning of the threshold at a constant value of 35 RFU.

The Ct values for each PCR reaction corresponding to sequence 2 (Tamra-Dabcyl probe) are determined after manual positioning of the baseline between cycles 4 and 30 and manual positioning of the threshold at a constant value of 20 RFU.

The Ct values for each PCR reaction corresponding to sequence 3 (Atto-590-Dabcyl probe) are determined after manual positioning of the baseline between cycles 4 and 30 and manual positioning of the threshold at a constant value of 10 RFU.

The means of the Ct values for the duplicates obtained after amplification of 10 copies/PCR and 1000 copies/PCR with the mixes (mixes 6 to 9) incubated at 37°C are shown in Figure 2.

Furthermore, similar experiments conducted with mix 10 incubated at 37°C and 4°C are presented in the following Table 5 for sequence 1, Table 6 for sequence 2, and Table 7 for sequence 3.

**Table 5: Ct values obtained from the amplification of DNA sequence 1 (Fam-Dabcyl probe), after various incubation times, at 37°C and 4°C, of PCR mix 10 containing 5 M glycerol + D.3 M PVP-10:**

| | **Ct (1,6.10² copies / PCR)** | | **Ct (1,6.10⁴ copies / PCR)** | |
|---|---|---|---|---|
| | **37°C** | **4°C** | **37°C** | **4°C** |
| **D0** | 35.4 | 35.4 | 28.0 | 28.0 |
| **D7** | 35.8 | NT | 25.9 | NT |
| **D14** | 35.9 | NT | 25.7 | NT |
| **D21** | 38.4 | NT | 26.1 | NT |
| **D28** | 43.9 | NT | 26.0 | NT |
| **6 months** | NT | 39.5 | NT | 28.2 |
| **12 months** | NT | 41.8 | NT | 31.2 |

**Table 6: Ct values obtained from the amplification of DNA sequence 2 (Tamra-Dabcyl probe), after various incubation times, at 37°C and 4°C, of PCR mix 10 containing 5 M glycerol + 0.3 M PVP-10:**

| | **Ct (10 copies / PCR)** | | **Ct (10³ copies / PCR)** | |
|---|---|---|---|---|
| | **37°C** | **4°C** | **37°C** | **4°C** |
| **D0** | 46.0 | 46.0 | 38.2 | 38.2 |
| **D7** | 44.4 | NT | 35.7 | NT |
| **D14** | 43.7 | NT | 35.8 | NT |
| **D21** | 44.4 | NT | 36.3 | NT |
| **D28** | > 50 | NT | 37.4 | NT |
| **6 months** | NT | > 50 | NT | 39.6 |
| **12 months** | NT | > 50 | NT | 38.3 |

**Table 7: Ct values obtained from the amplification of DNA sequence 3 (Atto-590-Dabcyl probe), after various incubation times, at 37°C and 4°C, of PCR mix 10 containing 5 M glycerol + 0.3-M PVP-10:**

| | **Ct (10⁴ copies / PCR)** | |
|---|---|---|
| | **37°C** | **4°C** |
| **D0** | 35.11 | 35.11 |
| **D7** | 34.94 | NT |
| **D14** | 35.51 | NT |
| **D21** | 36.08 | NT |
| **D28** | 38.91 | NT |
| **6 months** | NT | 37.2 |
| **12 months** | NT | 36.8 |

Glycerol or PVP-10 makes it possible to stabilize a mix for real-time PCR containing several fluorescent probes.

The combining of PVP-10 and glycerol makes it possible to increase this stability.

### Example 3:

This example compares the stability of the same composition conserved at three different temperatures: 37°C, 20°C and 4°C.

A composition for real-time PCR is prepared. It contains 2X buffer (Qiagen), 3 mM of MgCl₂, 6 µM of each primer (Eurogentec), 2 mM of dATP, 2 mM of dGTP, 2 mM of dCTP, 2 mM of dUTP, 1 mM of dTTP (Amersham), 6 µM of Molecular Beacon probe labelled with Fam-Dabcyl (Eurogentec), 2.5 U of Taq polymerase (Qiagen), 0.25 U of UDG (Invitrogen), 6.5M glycerol (Prolabo) and 300 mM PVP-10 (Sigma).

Aliquot fractions of 45 µl are taken from each composition (mix) and conserved at 37°C, at 20°C or at 4°C until the date of analysis.

A diluent solution is prepared. It contains 2X buffer and 14.25 mM of MgCl₂. Aliquot fractions of 180 µl are taken and conserved at 37°C, at 20°C or at 4°C until the date of analysis.

On the day of analysis, an aliquot fraction of the diluent is added to an aliquot fraction of the PCR mix. After mixing, 25 µl of the solution are dispensed into the PCR tubes.

25 µl of hepatitis B virus DNA containing 1 copy/µl are added to two different PCR tubes, 25 µl of hepatitis B virus DNA containing 10 copies/µl are added to two different tubes, 25 µl of hepatitis B virus DNA containing 100 copies/µl are added to two different tubes and 25 µl of water are added to two different tubes.

PCR reactions are carried out on day 0, on day 21, on day 35 and then every month between 2 and 6 months, at 9 months and at 12 months.

The PCR reactions are carried out on the iQ icycler (Bio-Rad). 50 cycles made up of 15 seconds at 95°C, 30 seconds at 55°C and 30 seconds at 72°C are carried out, after an initial step of 10 minutes at 37°C in order to activate the UDG and of 15 minutes at 95°C in order to activate the "hot start" polymerase.

The analyses are carried out by means of the iQ icycler software. The Ct values for each PCR reaction are determined after manual positioning of the baseline between cycles 2 and 32 and manual positioning of the threshold at a constant value of 50 RFU.

The means of the Ct values for the duplicates obtained after amplification of 25 copies/PCR, 250 copies/PCR and 2500 copies/PCR of the composition conserved at 37°C, 20°C and 44°C are shown in Table 8 below:

**Table 8: Comparison of the Ct values obtained for the amplification of 25 copies of DNA, 250 copies of DNA and 2500 copies of DNA after various conservation times of the composition, at 37°C, at 20°C and at 4°C**

| | **Ct (25 copies/PCR)** | | | **Ct (250 copies/PCR)** | | | **Ct (2500 copies/PCR)** | | |
|---|---|---|---|---|---|---|---|---|---|
| | **37°C** | **20°C** | **4°C** | **37°C** | **20°C** | **4°C** | **37°C** | **20°C** | **4°C** |
| **D0** | 41.0 + 0.7 | 41.0 + 0.7 | 41.0 + 0.7 | 37.4 + 0.0 | 37.4+0.0 | 37.4 + 0.0 | 34.7+0.0 | 34.7 + 0.0 | 34.7 + 0.0 |
| **D21** | 41.1 + 3.7 | 39.7 + 0.0 | 40.3 + 0.2 | 4.9 + 0.0 | 36.0 + 0.8 | 37.0 + 0.1 | 32.0 + 0.0 | 33.0 + 0.6 | 33.9 + 0.1 |
| **D35** | 48.4 + 1.61 | 40.0 + 0.2 | 41.2 + 0.3 | 34.6 + 0.6 | 35.0 + 0.7 | 36.3 + 0.0 | 30.4 + 0.3 | 32.7 + 0.0 | 33.4 + 0.3 |
| **2 months** | >50 | 41.2 + 44 | 42.1 + 0.7 | >50 | 36.5 + 0.0 | 37.2 + 0.4 | 45 + 1.2 | 33.4 + 0.1 | 34.5 + 0.6 |
| **3 months** | NT | 38.4 + 0.2 | 40.9 + 0.2 | NT | 35.0 + 0.2 | 36.8 + 0.3 | >50 | 32.4 + 0.1 | 33.3 + 0.1 |
| **4 months** | NT | 38.3 + 0.1 | 39.7 + 2.9 | NT | 35.4 + 0.5 | 36.8 + 0.0 | NT | 32.1 + 0.1 | 33.4 + 0.3 |
| **5 months** | NT | NT | 39.8 + 0.1 | NT | NT | 37.3 + 6.5 | NT | NT | 34.5 + 0.5 |
| **6 months** | NT | 40.6 + 0.7 | 40.0 + 0.6 | NT | 38.4 + 0.4 | 37.0 + 0.4 | NT | 34.6 + 0.2 | 34 + 0.4 |
| **9 months** | NT | >50 | 40.2 + 0.5 | NT | >50 | 36.8 + 0.2 | NT | >50 | 33.9 + 0.2 |
| **12 months** | NT | NT | 42 + 0.7 | NT | NT | 37.5 + | 0.4 NT | NT | 34.9 + 0.5 |

A composition that is stable for 21 days at 37°C is stable for at least 6 months at ambient temperature and at least 12 months at 4°C.

In conclusion, all the results presented demonstrate a stability of the compositions of greater than. 6 months at 20°C, and greater than 1 year at 4°C, and a longer stability at -20°C.

### BIBLIOGRAPHY

- Cayouette M, Sucharzuk A, Moores J, Tyagi S, and Kramer FR (1999) Using molecular beacons to monitor PCR product formation. Strategies Newsl. 12:85-88.
- Fahy et al. (1991) PCR Meth. Appl., 1, 25-33
- Kwoh et al (1989) PNAS, 86, 1173-1177
- Saiki et al., (1988), Science, 239:487
- Tyagi S and Kramer FR (1996) Nature Biotechnol., 16, 303-308
- Walker et al. (1992) P.N.A.S, 89, 392-396

## Claims

1. A concentrated and buffered liquid composition for amplifying nucleic acids, comprising at least one dNTP, at least one enzyme required for the amplification, at least one oligonucleotide primer, at least one fluorescent nucleotide probe, and polyvinylpyrrolidone (PVP).

2. A composition according to claim 1, further comprising at least one polyol.

3. A composition according to claim 2, in which the polyol is selected from glycerol, sorbitol, inositol and pentaerythritol.

4. A composition according to any one of claims 1 to 3, further comprising a magnesium salt or a manganese salt.

5. A composition according to any of claims 1 to 4, for amplifying nucleic acids by PCR, comprising dATP, dCTP, dGTP, and one among dTTP or dUTP, and also at least one enzyme required for the PCR, at least two oligonucleotide primers, and at least one fluorescent nucleotide probe.

6. A composition according to any of claims 1 to 4, for amplifying nucleic acids by RT-PCR, comprising dATP, dCTP, dGTP, and one among dTTP or dUTP, and also at least one enzyme required for the RT-PCR, at least two oligonucleotide primers, and at least one fluorescent nucleotide probe.

7. A composition according to claim 3, comprising at least 5M of glycerol.

8. A composition according to claim 3, comprising at least 1 M of sorbitol.

9. A composition according to claim 3, comprising at least 500 mM of inositol.

10. A composition according to claim 3, comprising at least 250 mM of pentaerythritol.

11. A composition according to any of claims 1 to 10, in which the PVP is PVP-10.

12. A composition according to claim 11, comprising at least 30 mM of PVP-10.

13. A method of preparing a complete reaction mix for amplifying nucleic acids, comprising:
(i) diluting a concentrated composition according to any of claims 1 to 12, in a buffered saline solution suitable for amplifying nucleic acids;
(ii)bringing a sample of nucleic acids to be amplified into contact with the desired volume of the composition diluted in step (i).

14. A method of preparing a complete reaction mix for amplifying nucleic acids, comprising:
(i) mixing a sample of nucleic acids to be amplified, in a buffered saline solution suitable for amplifying nucleic acids;
(ii) adding the mix obtained in step (i) to a desired volume of the concentrated composition according to any of claims 1 to 12.

15. A method of amplifying nucleic acids, comprising steps (i) and (ii) as defined in claim 13 or 14, and (iii) the starting of the reaction for amplifying the nucleic acids present in the sample, which amplification is detectable by means of the fluorescent label carried by the probe.

16. A method of amplification according to claim 15, which is a real-time RT-PCR.

17. A method of amplification according to claim 15, which is a real-time PCR.

18. A kit for amplifying and/or detecting nucleic acids, comprising at least one container containing the concentrated composition as defined in any of claims 1 to 12.

19. A kit for amplifying and/or detecting nucleic acids according to claim 18, comprising a first container containing the concentrated composition as defined in any of claims 1 to 12, and which further comprises a second container containing a buffered saline solution suitable for amplifying nucleic acids.

20. A kit according to claim 19, in which the buffered saline solution contains at least one of the ingredients selected from TRIS at a concentration of at least 8 mM, potassium salt or sodium salt at a concentration of at least 8 mM, ammonium salt at a concentration of at least 5 mM, and magnesium salt or manganese salt at a concentration of at least 0.8 mM, the ingredients being used alone or as a mixture.

21. The use of polyvinylpyrrolidone (PVP), and optionally of a polyol, for stabilizing both enzymes and fluorescent nucleotide probes in a liquid composition.

22. The use according to claim 21, in which the PVP, and optionally the polyol, also stabilize(s) dNTPs.

23. The use according to claim 21 or 22, in which the liquid composition is a concentrated and buffered composition for amplifying nucleic acids, comprising at least one dNTP, at least one enzyme required for the amplification, at least one oligonucleotide primer, and at least one fluorescent nucleotide probe.

24. The use according to any of claims 21 to 23, in which the polyol is selected from glycerol, sorbitol, inositol and pentaerythritol.

25. The use according to any of claims 21 to 23, in which the PVP is PVP-10.

## Patentansprüche

1. Eine konzentrierte und gepufferte, flüssige Zusammensetzung zur Amplifizierung von Nukleinsäuren, aufweisend mindestens ein dNTP, mindestens ein zur Amplifizierung benötigtes Enzym, mindestens einen Oligonukleotid-Primer, mindestens eine fluoreszierende Nukleotidsonde und Polyvinylpyrrolidon (PVP).

2. Eine Zusammensetzung nach Anspruch 1, die ferner mindestens ein Polyol aufweist.

3. Eine Zusammensetzung nach Anspruch 2, in welcher das Polyol aus einer Gruppe ausgewählt wird, die Glycerol, Sorbitol, Inositol und Pentaerythritol aufweist.

4. Eine Zusammensetzung nach einem der Ansprüche 1 bis 3, die ferner ein Magnesiumsalz oder ein Mangansalz aufweist.

5. Eine Zusammensetzung nach einem der Ansprüche 1 bis 4 zur Amplifizierung von Nukleinsäuren durch PCR, aufweisend dATP, dCTP, dGTP, und eines von dTTP oder dUTP, und auch mindestens ein zur PCR benötigtes Enzym, mindestens zwei Oligonukleotid-Primer, und mindestens eine fluoreszierende Nukleotidsonde.

6. Eine Zusammensetzung nach einem der Ansprüche 1 bis 4 zur Amplifizierung von Nukleinsäuren durch RT-PCR, aufweisend dATP, dCTP, dGTP, und eines von dTTP oder dUTP, und auch mindestens ein zur RT-PCR benötigtes Enzym, mindestens zwei Oligonukleotid-Primer, und mindestens eine fluoreszierende Nukleotidsonde.

7. Eine Zusammensetzung nach Anspruch 3, die mindestens 5 M Glycerol aufweist.

8. Eine Zusammensetzung nach Anspruch 3, die mindestens 1 M Sorbitol aufweist.

9. Eine Zusammensetzung nach Anspruch 3, die mindestens 500 mM Inositol aufweist.

10. Eine Zusammensetzung nach Anspruch 3, die mindestens 250 mM Pentaerythritol aufweist.

11. Eine Zusammensetzung nach einem der Ansprüche 1 bis 10, in welcher das PVP ein PVP-10 ist.

12. Eine Zusammensetzung nach Anspruch 11, die mindestens 30 mM PVP-10 aufweist.

13. Ein Verfahren zur Anfertigung eines kompletten Reaktionsgemisches zur Amplifizierung von Nukleinsäuren, aufweisend:
(i) Verdünnen einer konzentrierten Zusammenstellung gemäß einem der Ansprüche 1 bis 12 in einer gepufferten, salzigen, zur Amplifizierung von Nukleinsäuren geeigneten Lösung;
(ii) in Kontakt bringen einer Probe einer zu verstärkenden Nukleinsäure mit dem gewünschten Volumen der in Schritt (i) verdünnten Zusammenstellung.

14. Ein Verfahren zur Anfertigung eines kompletten Reaktionsgemisches zur Amplifizierung von Nukleinsäuren, aufweisend:
(i) Mischen einer Probe einer zu verstärkenden Nukleinsäure in einer gepufferten, salzigen, zur Amplifizierung von Nukleinsäuren geeigneten Lösung;
(ii) Hinzufügen der in Schritt (i) erhaltenen Mischung zu einem gewünschten Volumen der konzentrierten Zusammensetzung gemäß einem der Ansprüche 1 bis 12.

15. Ein Verfahren zur Amplifizierung von Nukleinsäuren, aufweisend Schritte (i) und (ii), wie in Anspruch 13 oder 14 definiert, und
(iii) Einleiten der Reaktion zur Amplifizierung der in der Probe vorhandenen Nukleinsäuren, wobei die Reaktion mittels der fluoreszierenden Marker, die von der Sonde getragen werden, nachweisbar ist.

16. Ein Verfahren zur Amplifizierung nach Anspruch 15, welches eine Echtzeit-RT-PCR ist.

17. Ein Verfahren zur Amplifizierung nach Anspruch 15, welches eine Echtzeit-PCR ist.

18. Ein Kit zur Amplifizierung und/oder Detektion von Nukleinsäuren, aufweisend mindestens einen die konzentrierte Zusammenstellung gemäß einem der Ansprüche 1 bis 12 enthaltenden Behälter.

19. Ein Kit zur Amplifizierung und/oder Detektion von Nukleinsäuren nach Anspruch 18, aufweisend einen die konzentrierte Zusammenstellung gemäß einem der Ansprüche 1 bis 12 enthaltenden ersten Behälter und ferner einen zweiten Behälter aufweisend, der eine gepufferte, salzige, zur Amplifizierung von Nukleinsäuren geeignete Lösung enthält.

20. Ein Kit nach Anspruch 19, in welcher die gepufferte salzige Lösung mindestens ein Bestandteil aus Gruppe enthält, die TRIS bei einer Konzentration von mindestens 8 mM, Kaliumsalz oder Natriumsalz bei einer Konzentration von mindestens 8 mM, Ammonium-Salz bei einer Konzentration von mindestens 5 mM, und Magnesiumsalz oder Mangansalz bei einer Konzentration von mindestens 0.8 mM enthält, wobei die Bestandteile gesondert oder als Mischung verwendet werden.

21. Die Verwendung von Polyvinylpyrrolidon (PVP) und wahlweise von Polyol zur Stabilisierung von sowohl Enzymen als auch fluoreszierender Nukleotidsonden in einer flüssigen Zusammenstellung.

22. Die Verwendung nach Anspruch 21, bei welcher das PVP und wahlweise das Polyol auch dNTP stabilisiert bzw. stabilisieren.

23. Die Verwendung nach Anspruch 21 oder 22, bei welcher die flüssige Zusammenstellung eine konzentrierte und gepufferte Zusammenstellung zur Amplifizierung von Nukleinsäuren ist, aufweisend mindestens ein dNTP, mindestens ein zur Amplifizierung benötigtes Enzym, mindestens einen Oligonukleotid-Primer, und mindestens eine fluoreszierende Nukleotidsonde.

24. Die Verwendung nach einem der Ansprüche 21 bis 23, bei welcher das Polyol aus einer Gruppe ausgewählt wird, die Glycerol, Sorbitol, Inositol und Pentaerythiritol aufweist.

25. Die Verwendung nach einem der Ansprüche 21 bis 23, bei welcher das PVP ein PVP-10 ist.

## Revendications

1. Composition liquide concentrée et tamponnée pour l'amplification d'acides nucléiques, comprenant au moins un dNTP, au moins une enzyme requise pour l'amplification, au moins une amorce oligonucléotidique, au moins une sonde nucléotidique fluorescente, et du polyvinylpyrrolidone (PVP).

2. Composition selon la revendication 1, comprenant au moins un polyol.

3. Composition selon la revendication 2, dans laquelle le polyol est sélectionné parmi le glycérol, le sorbitol, l'inositol et le pentaérythritol.

4. Composition selon l'une quelconque des revendications 1 à 3, comprenant en outre un sel de magnésium ou un sel de manganèse.

5. Composition selon l'une quelconque des revendications 1 à 4, pour l'amplification d'acides nucléiques par PCR, comprenant dATP, dCTP, dGTP, et un parmi dTTP ou dUTP, et également au moins une enzyme requise pour la PCR, au moins deux amorces oligonucléotidiques, et au moins une sonde nucléotidique fluorescente.

6. Composition selon l'une quelconque des revendications 1 à 4, pour l'amplification d'acides nucléiques par RT-PCR, comprenant dATP, dCTP, dGTP, et un parmi dTTP ou dUTP, et également au moins une enzyme requise pour la RT-PCR, au moins deux amorces oligonucléotidiques, et au moins une sonde nucléotidique fluorescente.

7. Composition selon la revendication 3, comprenant au moins 5 M de glycérol.

8. Composition selon la revendication 3, comprenant au moins un 1 M de sorbitol.

9. Composition selon la revendication 3, comprenant au moins 500 mM d'inositol.

10. Composition selon la revendication 3, comprenant au moins 250 mM de pentaérythritol.

11. Composition selon l'une quelconque des revendications 1 à 10, dans laquelle le PVP est du PVP-10.

12. Composition selon la revendication 11, comprenant au moins 30 mM de PVP-10.

13. Procédé de préparation d'un mélange réactionnel complet pour l'amplification d'acides nucléiques, comprenant les étapes consistant à :
(i) diluer une composition concentrée selon l'une quelconque des revendications 1 à 12, dans une solution saline tamponnée convenant pour l'amplification d'acides nucléiques;
(ii) mettre un échantillon d'acides nucléiques à amplifier en contact avec le volume souhaité de la composition diluée dans l'étape (i).

14. Procédé de préparation d'un mélange réactionnel complet pour l'amplification d'acides nucléiques, comprenant les étapes consistant à :
(i) mélanger un échantillon d'acide nucléique à amplifier dans une solution saline tamponnée convenant pour l'amplification d'acides nucléiques ;
(ii) ajouter le mélange obtenu dans l'étape (i) à un volume souhaité de la composition concentrée selon l'une quelconque des revendications 1 à 12.

15. Procédé d'amplification d'acides nucléiques, comprenant les étapes (i) et (ii) telles que définies dans la revendication 13 ou 14, et (iii) le démarrage de la réaction destinée à l'amplification des acides nucléiques présents dans l'échantillon, cette amplification étant détectable au moyen du marquage fluorescent transporté par la sonde.

16. Procédé d'amplification selon la revendication 15, qui est une RT-PCR en temps réel.

17. Procédé d'amplification selon la revendication 15, qui est une PCR en temps réel.

18. Trousse pour l'amplification et/ou la détection d'acides nucléiques, comprenant au moins un conteneur contenant la composition concentrée telle que définie dans l'une quelconque des revendications 1 à 12.

19. Trousse pour l'amplification et/ou la détection d'acides nucléiques selon la revendication 18, comprenant un premier conteneur contenant la composition concentrée telle que définie dans l'une quelconque des revendications 1 à 12, et comprenant en outre un second conteneur contenant une solution saline tamponnée convenant pour l'amplification d'acides nucléiques.

20. Trousse selon la revendication 19, dans laquelle la solution saline tamponnée contient au moins un des ingrédients sélectionnés parmi du TRIS en une concentration d'au moins 8 mM, du sel de potassium ou du sel de sodium en une concentration d'au moins 8 mM, du sel d'ammonium en une concentration d'au moins 5 mM, et du sel de magnésium ou du sel de manganèse en une concentration d'au moins 0,8 mM, les ingrédients étant utilisés seuls ou sous forme d'un mélange.

21. Utilisation de polyvinylpyrrolidone (PVP), et facultativement d'un polyol, pour la stabilisation à la fois des enzymes et des sondes nucléotidiques fluorescentes dans une composition liquide.

22. Utilisation selon la revendication 21, dans laquelle le PVP, et facultativement le polyol, stabilise(nt) également des dNTP.

23. Utilisation selon la revendication 21 ou 22, dans laquelle la composition liquide est une composition concentrée et tamponnée pour l'amplification d'acides nucléiques, comprenant au moins un dNTP, au moins une enzyme requise pour l'amplification, au moins une amorce oligonucléotidique, et au moins une sonde nucléotidique fluorescente.

24. Utilisation selon l'une quelconque des revendications 21 à 23, dans laquelle le polyol est sélectionné parmi le glycérol, le sorbitol, l'inositol et le pentaérythritol.

25. Utilisation selon l'une quelconque des revendications 21 à 23, dans laquelle le PVP est du PVP-10.
